(19) Europäisches Patentamt
European Patent Office
Office européen des brevets

(11) **EP 2 695 850 A1**

## EUROPEAN PATENT APPLICATION
published in accordance with Art. 153(4) EPC

(43) Date of publication:
**12.02.2014 Bulletin 2014/07**

(21) Application number: **11850247.5**

(22) Date of filing: **16.12.2011**

(51) Int Cl.:
*B82Y 40/00* (2011.01)   *C04B 24/02* (2006.01)
*C08K 5/053* (2006.01)   *E21D 5/04* (2006.01)
*A61K 6/06* (2006.01)   *E04C 3/00* (2006.01)

(86) International application number:
**PCT/BR2011/000484**

(87) International publication number:
**WO 2012/083400 (28.06.2012 Gazette 2012/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2010 BR PI1009165**

(71) Applicant: **Inovamat- Inovação Em Materiais LTDA Empresa Brasileira**
**São Carlos - São Paulo CEP 13571-000 (BR)**

(72) Inventors:
• **MOREIRA, Thatiana Andressa**
**CEP 13562-002 (BR)**
• **ROSSETTO, Herbert Luis**
**CEP 13562-002 (BR)**

(74) Representative: **Dinis, Daniela**
**Avenida Almirante Reis, N° 123, 1° F**
**1169-157 Lisboa (PT)**

(54) **NUCLEATION PROCESS AND IN SITU GROWTH OF NANOMETRIC CALCIUM SILICATE-BASED CRYSTALS IN CEMENT MATERIALS, CALCIUM SILICATE-BASED NANOMETRIC CHRYSTALS, USE OF CHRYSTALS AND USE OF GLYCERIN**

(57)    This invention refers to the nucleation and growth process of calcium silicate-based nanocrystals and the toughening of cement materials resulting from the in situ growth of these respective crystals along the hydration reaction in cement, preferentially Portland cement, or another one with similar composition, for several applications, among them the increase in mechanical performance of cement materials for: the filling of underground space between the geological formation and the duct for petroleum or gas exploration in petrochemical wells; the filling of odontological and orthopedical cavities; and the conformation of structural elements for civil work.

FIG. 1

## Description

### Invention Field

This invention refers to the nucleation process and growth of calcium silicate-based nanocrystals and the toughening of cement materials resulting from in situ growth of these respective crystals along the hydration reaction in the cement, preferentially Portland cement or other one with similar composition, for several applications, among them the increase in the mechanical performance of cement materials to: filling of the underground space between the geological formation and the petroleum or gas exploration duct in petrochemical wells; the filling of odontological and orthopedical cavities; and the conformation of structural elements for civil work.

### Background of the invention

The cement is certainly the most consumed material by the human kind, mainly in civil work activities, for whose application Portland cement stands out, although also playing an important role in biomedical applications and in petrochemical well cementation. In fact, the cementation of petrochemical wells, is probably, the application to which the cement plays the most critical role in terms of investment return. In other words, the petrochemical well cementation is one of the most important activities for the success of its drilling and for longevity of its operation. The importance is such that any type of carelessness over the quality control of cement paste which covers the annular space between the duct and geological formation is unacceptable, besides its physical integrity, under the penalty of jeopardizing the investment of million of dollars that usually features the scale for drilling costs and, also, condemn the well then drilled to extinction, what  represents additional costs. The depletion of oil and gas sources in the planet has impelled the exploration of hydrocarbons to the engineering limits and such trend should continue thanks to the following statistics: the emerging countries comprising the group called BRIC, that is, Brazil, Russia, India and China, comprise about half of the world population, however, they consume only about 20% of the whole energy potential being explored in the planet. It means that within the next 20 years the energy potential shall be increased in 35% regarding the 2005 consumption level.

Although there are growing incentives for the "petroleum economy" transition to "bio-energy economy", that transition should be slight and continuous, due to the global energy matrix dependence to hydrocarbons, what represents a guarantee for exploration of reservoirs still being discovered. In general, the cement mixtures for petrochemical well are characterized, among other factors, by: (i) not segregating the liquid and solid phases when under pressure; (ii) avoiding the excessive loss of water when exposed to high temperatures; (iii) providing viscosity compatible to pumping; (iv) proper diffuse time - avoiding premature hardening and, at the same time, its excessive wait, due to the cost of equipment; (v) proving the duct protection against corrosion; and (vi) isolating ducts from geological formations to avoid contaminations and/or oil/gas loss. The first four are important factors during works with cement paste, while the last two ones guarantee the well useful life. It's at this point where the main challenges lie: meeting the needs of fluidity for cementation to requirements regarding the mechanical performance during the well operation. As illustrate, the cement paste used in the petrochemical well cementation is pumped into the annular space between the well geological formation and the external duct wall through where the hydrocarbon will be extracted from underground reservoir. That cement covering hardens in loco and positions the duct, bearing it along its length, besides avoiding the oil/gas migration to outside of the production area. However, the high mechanical resistance development by the cement covering is mandatory. Many are the mechanical requests to which the wells covering are submit to, that is: (i) if the pressure and/or temperature within the duct increases, the resulting internal pressure expands the radial duct and longitudinally, what induces to a tension in the cement adjacent covering, leading it to cracks or the rupture in the adhesion between both; (ii) another example is when confined fluids in cement covering thermally expands causing high internal pressures. That condition usually happens with fluids injection in high temperature, such as steam, for instance, for oil recovery in wells with natural decline of production; (iii) another condition includes the force applied by the dislocations of geological formation surrounding the covering; and, finally, (iv) impacts and/or chocks generated by drilling and other operations in the well. In all these conditions, the increase of mechanical resistance of cement paste is important.

In response to this challenge, the state of the technique, according to the inventors' knowledge so far, has addressed the two requirements above - that is, the fluidity and the mechanical resistance - in an isolated way. The application of this North American patent US 2001/0004936 reveals a cement composition for cementation of petrochemical wells in presence of rubber aqueous latex and silicon oil coated silica, that results in a resilient solid. In turn, the patent application US 2001/0018973 describes cement composition able to be pumped to the well covering and, after hardening, resistant to fragmentation due to the presence of polypropylene fibers with hydrophilized surface. According to the invention teaching, the fibers restriction to the content of 1% in cement weight avoids a common problem to fiber addition to cement materials: agglomeration. On the other hand, patent US 7424914 demands the toughening of cement composition that covers the petrochemical wells by adding inorganic fibers  regarding that aspect, that is, length (lower than 10mm) regarding the diameter, over 25, being such fibers, preferentially, based on wollastonite, among other ceramics, in content from 0.1 to 20% in cement weight in the composition.

On the other hand, expansible cements have been recognized by the best results in the primary cementation of petrochemical wells, what represents lower expenditure of time and expenses from the reduction of its remediation. There is a gap between the current expansive cements in the market that will be filled by this invention. Among the cement formulations currently in use for such purpose: (i) Ettringite System - comprises most of the expansible cements for wells (among them the most known in the market, Microbond, supplied by Halliburton, USA) and is based on forming acicular ettringite crystals (calcium sulfoaluminate) at the end of the cement magpie. For such, calcium sulfate, at the rate from 10 to 15% in cement weight, is added to the cement rich in tricalcium aluminate. That results into the formation of ettringite crystals having density lower than those of the reagents forming them. With that, the expansion arises from the internal pressure applied by such crystallization. However, the highest limitation of that system is its expansion incapacity in a temperature over 76°C; (ii) Saline Systems - is among the first methods to reach a cement desired expansion: lineal expansion of up to 0.4%, similar to the ettringite one. The expansion cause is also similar to the ettringite one: internal pressure applied by the salt crystallization in pores. The system limitation is the mechanical resistance reduction, given by the adhesion reduction among cement particles which results into the dispute for hydrogen connections with confined water molecules [Rossetto, H.L., de Souza, M.F., and Pandolfelli, V.C., Chaotropic Substances and their Effects on the Mechanical Strength of Portland Cement-Based Materials, Mat. Ras., vol. 11, 183-85, 2008]; and (iii) MgO System - the magnesium oxide provokes the expansion within the cement matrix due to its hydration to magnesium hydroxide (brucite) that, in turn, occupies more space than the reagents that originated it, that is, MgO and water. For such, the magnesium oxide (MgO) should be burned between 1100 and 1300°C, otherwise, the reaction takes place before the magpie reaction and no expansion is observed. The systems containing MgO have shown excellent expansion (up to 2.0% lineally) even in high temperatures (288°C) with only 1% of addition in cement weight. However, in temperature below 60°C, the hydration reaction is slow in producing practical effects.

The addition of fibers with wollastonite-based high aspect ratio has also been reported as an efficient mechanism for toughening of cement pastes for conformation of structural elements of civil work (Low, N.M.P. & Beaudoin, J.J., The flexural toughness and ductility of Portland cement-based binders reinforced with wollastonite micro-fibers, Cem. Concr. Res., vol. 24 n.2, 250-58 1994): the resistance to flexion of cement materials doubles with the addition of wollastonite micro-fibers at the proportion from 2 to 15% in volume and increases from four to ten times when micro-silica is added together with the wollastonite micro-fibers, at the proportion from 5 to 17.6% in volume. Two will be the factors that will force the increment in the mechanical performance of Portland cement-base-materials in civil work: (i) the vertical occupation trend of big cities, bound to architectural challenges of modern construction, go to the slenderness of structural elements and, with that, a mechanical performance designed according to engineering of materials; and (ii) the increasing environmental appeal for reduction in gas generation which contribute to the global heating - the cement production, in amount, is the second biggest carbon gas source, since each ton of produced cement emits about a ton of carbon gas in atmosphere - what will demand the Portland cement use rationalization. Based on the growth forecasts in the cement global demand, estimate in 5% a year, the moment for such seems to have arrived.

In turn, the toughening of cements for biomedical applications has been promising (Müller, F.A., Gbureck, U., Kasuga, T., Mizutani, Y., Barralet, J.E., and Lohbauer, U., Whisker-reinforced calcium phosphate cements, J. Am. Ceram. Soc., vol. 90, n. 11, 3694-97, 2007): an increase of 60% in the resistance to compression and 122% in the fracture energy was reported to calcium phosphate cement from the addition of hydroxiapatite crystals in the proportion of 30% in volume. Although the calcium phosphate is the most usual option as a cement for biomedical applications, due to its live re-absorption, in fact the option for other cements with low or no bio-reabsorption is feasible since the material is biocompatible and presents long-term enough mechanical resistance for a permanent application. The US 5769638 patent claims a composition based on Portland cement for filling cavities in the teeth's inner parts, specialty known as endodontics, with results allowing the prophylaxis of clinical cases before condemnable to toot pulling out. Recently, the speculation on the use of calcium silicates for filling bone cavities proved the material bio-compatibility, be in form of gel, which represents partially the cement micro-structural organization (Su, J., Wang, Z., Yonggang, Y., Yongfa, W., Cao, L., Ma, Y., Yu, B., and Li, M., Nanoporous calcium silicate and PLGA bio-composite for bone repair, J. Nanomaterials, 181429 2010), be as crystals, as polymeric matrix mechanical reinforcement (Rodriguez-Lorenzo, L.M., Garcia-Carrodeguas, R., Rodriguez, M.A., de Aza, S., Jiménez, J., López-Bravo, A., Fernandez, M., and San Román, J., Synthesis, characterization, bioactivity and biocompatility of nanostructured materials based on the wollastonite-poly (ethylmethacrylate-co-vynilpyrrolidone) system, J. Biomol. Mater. Res. A, vol. 88, 53-64, 2008), what tends to reproduce the natural anisotropy of bone tissue, that is, the variation in mechanical properties according to the change toward and in the sense of active force. The polymethylmethacrylate, or PMMA, is the most used cement currently for filling bone faults, comprising a monomer with strong odor and causes high heat generation at the end of its polymerization: temperature of 130°C. Among other side effects, PMMA may cause: necrosis of adjacent tissues, mainly spinal nerves, when applied in vertebroplasty procedures - depending on the volume of material used and the proximity to tissues; pulmonary embolism; stroke and heart attack (when in contact with blood current); and osteopenia, that is, decrease of bone mineral density due to the unbalance of tensions resulting from difference among PMMA's and bone elastic modules, reminding that the bone has piezoelectric nature responsible for the maintenance of ion calcium in its structure. Based on the demand of clinical cases whose benefits of the current treatments, in deontology as well as in orthopedics, are included in the life expectation increase of the planet's population, certainly there is a niche for the inventive activity comprising this invention.

It should be pointed out that wollastonite is a calcium silicate-based mineral, with the same chemical nature of Portland cement, and crystals with length between 4 and $80\mu m$ and diameter lower than $10\mu m$, whose industrial suppliers are already well established in global scale. The obtaining of that inorganic material by chemical route has been reproduced successfully (Lin, K., Chang, J., and Lu, J., Synthesis of wollastonite nanowires through hydrothermal microemulsion methods, Mater. Lett., vol. 60, 3007-10, 2006; Lin, K., Chang, J., Chen, G., Ruan, M., and Ning, C., Simple method to synthesize single-crystalline ($\beta$-wollastonite nanowires, J. Crystal Growth, vol. 300, 267-71, 2007), although with the aid of autoclaves and, in some cases, coadjuvants to coordinate the nucleation and the growth of crystals. Those processes result in nanometric crystals which favor the toughening of materials, among them cement, from very much low contents of addition in comparison to the mineral micrometric crystals  effects. However, micrometer scale reduction for nanometer can associate to crystals the same occupational health problem connected to asbestos, a fiber also inorganic and with high potential of toughening of cement materials whose use was abolished due to silicone induction, degenerative disease to lung functionality.

Therefore, the state of the technique still lacks a process able to induce forming those crystals that toughens the cement materials from the nucleation and growth of those respective in situ crystals and along the own hydration in cement, without impairment top the green rheology to green, a term used for reference to the workable state of cement paste, and, at the same time, without problems related to occupational health.

**Brief Description of the Figures**

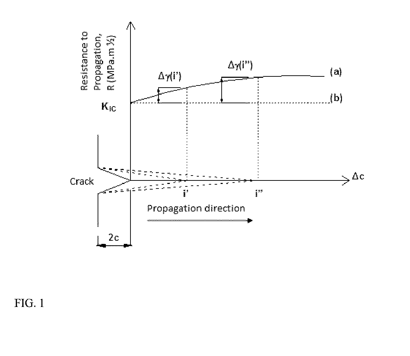

Figure 1 shows resistance to propagation (R) of a crack (with 2c length) while it spreads ($\Delta c$) : (a) when $\Delta\gamma \# 0$, the material may present the behavior of a growing 'Curve-R'; (b) whereas $\Delta\gamma = 0$, the material doesn't present additional difficulties against the crack propagation, or, in other words, it presents flat 'Curve-R.'

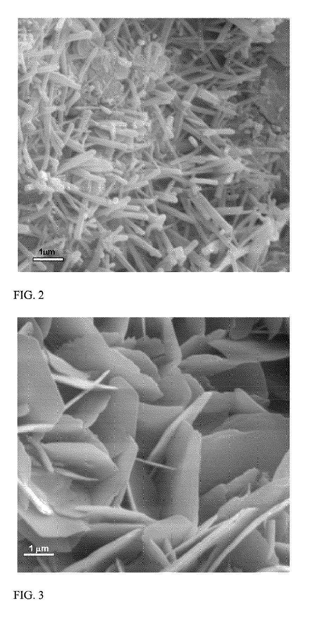

Figure 2 shows acicular nanometric crystals, nucleated under glycerin action in 1% concentration in cement weight in wetting water and grown in situ on the surface of cement grains, along the hydration reaction, able to toughen the referred cement material, that is, to increase the resistance to propagation of cracks that would lead to its rupture. Composition of nanocrystals: calcium silicate.

Figure 3 shows nanometric and lamelar crystals, nucleated under glycerin action in 10% concentration in cement weight in wetting water and added in situ on the surface of cement grains, along the hydration reaction, able to toughens the referred cement material, that is, to increase the resistance to propagation of cracks that would lead to its rupture. Composition of nanocrystals: calcium  silicate.

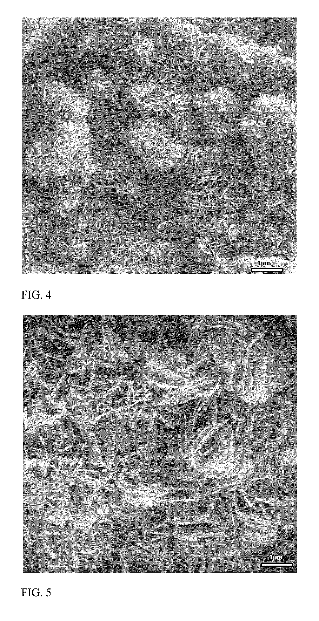

Figure 4 shows the reproduction of nucleation process and in situ growth of calcium silicate-based nanocrystals in cement materials for relation extreme condition in weight of water/cement (a/c) = 100.

Figure 5 shows the stability of nucleated calcium silicate-based nanocrystals, and increased in situ to cement paste, in other words, resistance to thermal decomposition, in temperature of 900°C for 24 hours.

**Invention Description**

Cements are rather cheap materials and available in global scale, but, due to the large use - it's considered the most used material in human kind - thanks to the fact of developing satisfactory mechanical resistance, under the structural standpoint, just by the reaction with water. For that reason, the cements are said to be hydraulic agglomerating and, among the cements, the one of larger use is the Portland type, whose composition is based on calcium silicates and aluminates which are moisturized spontaneously in contact with water, what results into its hardening and in resistance gain.

The resistance to compression is by far the most important cement mechanical property, which, on the other hand, is considered fragile regarding the traction forces requests in flexion and, for that reason receive such reinforcements such as short or long fibers.

Previous assessments show that such fragility is not inherent to the material but to a consequence of processing responsible for conforming the Portland cement-based products. Ever since, it has been endeavored for the resistance increase to flexion of cement materials, being the addition of fibers a traditionally incurred mechanism. However, favorable results depend on the fiber addition content, usually high, and on the mixture energy for dispersion of such fibers in the cement matrix, so that there is no impairment to the rheology of  material still in workable state, also called green, and to the own resistance to compression.

An alternative would be the fiber size reduction of the traditional micrometric scale for nanometric dimensions, what bumps into the economical feasibility and on the suspicion of occupational diseases associated to the handling of these nano-materials along the processing of cement materials.

The mechanism through which the fibers toughen the ceramic materials in general, and, among them, the cement materials, takes place by the deflection on the way to the propagation of cracks and also by the anchorage of surfaces so fractured.

It's understood by tenacity the energetic concept for fracture of a material or, in other words, how much energy a material can absorb before its fracture. All material has a characteristic rate ($K_{IC}$) related to its resistance to nucleation and the propagation of a crack, which, in energetic terms, can be set out in the following way:

$$K_{IC} = \sqrt{E2\gamma} = \sigma\sqrt{\pi c} \tag{1}$$

The equation above shows that the resistance to nucleation and to propagation ($K_{IC}$) of a crack depends basically on the material elastic module (E) and on the energy for creation of two new surfaces ($2\gamma$) from the crack propagation. Such material property can be measured through macroscopic parameters such as the resistance to traction of material ($\sigma$) and the crack critical size (c), which can be considered as the entity that most concentrates tension in the material, in general, a fault inherent to micro-structure, more commonly a pore. For the simple rearrangement of the equation above, two new concepts emerge, as shown in the following equation:

$$2\gamma = \pi c\sigma^2 / E \tag{2}$$

To the left of the equality in equation 2, the resistant (R) component represents the energetic cost to create two new surfaces by the fracture, and, to the right, the rate of stored elastic energy release (G). The imminence for crack propagation is represented by the condition R=G, or $K_{IC}$, however, once the crack propagation has started, what matters is if the energy for the material fracture or, simply, tenacity, what represents the material capacity presenting additional difficulties to the propagation of that crack ($\Delta\gamma$), in addition to the energy to create two new surfaces ($2\gamma$). In energetic terms, that defines the concept of fracture energy, $\gamma WOF = 2\gamma + \Delta\gamma$ represented graphically by Figure 1. In Figure 1, if the resistance to crack propagation (R) increases while the crack spreads ($\Delta c$), what is called behavior of growing Curve-R, it's a sign of an ongoing toughening mechanism or $\Delta\gamma \neq 0$, for instance, by the presence of fibers, as mentioned above; otherwise, the material doesn't show additional difficulties to crack propagation or $\Delta\gamma = 0$ what's called behavior of flat Curve-R.

This invention refers to the toughening of cement materials from a process that allows the nucleation and the growth of nanometric crystals in the own cement paste, reason why the term in situ is used along its hydration reaction, from the addition of an organic substance to wetting water: glycerin.

Also known as glycerol or also propane-1,2,3-triol, glycerin is an alcohol trivalent aliphatic of molecular formula $C_3H_8O$ that is shown in a liquid form in room temperature. Glycerin is present in all oils and fats of vegetable and animal origin, and by the way it's obtained - usually results as a by-product from the manufacturing of several products such as polypropylene, fatty acids and, ultimately, biodiesel - has low cost. Without regarding the theory, glycerin acts as surfactant of ions that result from the dissolution of anhydrous particles of Portland cement in hydration, mainly the calcium ($Ca^{2+}$) silicate ($SiO_4^{4-}$) and hydroxyl (OH -, responsible for the peculiar alkalinity to hydration of Portland cement). In those conditions, glycerin assumes a morphologic configuration that depends on its concentration in alkaline ionic solution, and that, by the adsorption of ions to their hydrophilic radicals (-OH), allows the precipitation of nanometric crystals of calcium silicate, in room temperature, that can assume acicular or lamellar habit. Acicular habit results from glycerin, in wetting water, in concentration from 0.5% to 2.5% in relative weight to the Portland cement weight, preferably 1% in weight relative to Portland cement, according to Figure 2, whereas the lamellar habit results from glycerin, in wetting water in content from 7.5% to 12.5% in weight regarding Portland cement weight, preferably 10% in weight regarding Portland cement weight, as shows in Figure 3. contents over 12.5% of glycerin in weight in wetting water, regarding the Portland cement weight, tend to significantly increase the viscosity of cement paste and, this way, interfere in the conformation capacity by conventional techniques. Consequently, the growth of these in situ nanocrystals provokes the volumetric expansion of cement material of up to 5% for acicular crystals and of more than 5% for lamellar ones, at the end of the hydration. Those calcium silicate-based nanometric crystals are stable, that is, are not decomposed even in temperatures of up to 900°C and toughen the Portland cement-based materials, that is, they provide such materials with a growing Curve-R behavior, what is shown by the increase in fracture energy and, further, by the increase of resistance to flexion: 100% for acicular crystals and over 200% for lamellar crystals, since the volumetric expansion can be controlled in the last case. The aspect relation of those nanocrystals, that is, relation between higher and lower dimension, is at least 10 and the precipitation and the growth of those crystals along the in situ hydration in the cement paste don't interfere to the rheology. The in situ precipitation and growth of calcium silicate-based nanocrystals according to this invention, in addition to providing a better mechanical performance when compared to mineral crystals, is exempted from the occupational health problems related to the toughening process by the addition of nanocrystals to the cement paste.

Thus, in a first aspect, this invention refers to in situ nucleation process and growth of calcium silicate-based nanometric crystals in cement materials, particularly of Portland type, that consists of including glycerin (particularly in aqueous solution) in cement in concentration from 0.5% to 12.5% in weight, in relation to the cement weight, maintaining relation in water weight for cement between 0.15 and 0.45. In a particular way, glycerin is used in liquid state and at room temperature.

The mixture is subject to the conformation with compacting aid whose pressure may vary from atmospheric up to 20MPa. The glycerin mixture to cement is accomplished by the aspersion or leaking of its aqueous solution on the powder or by the addition of powder to the aqueous solution, particularly with the aid of mechanical equipment.

The conformation of the obtained cement material can be accomplished by the procedures known of the technique, for instance, pressing, pumping, injection, extrusion, extrusion sliding forms or the action of flowing in molds or cavities, with or without the vibration aid.

In a particular way, additives are added to accelerate the cement hardening selected from:

-　calcium aluminate and/or its respective cements, in content from 0 to 30% in weight regarding the cement weight;
-　organic acids, such as citric, tartaric or oxalic, among others, and its respective salts, in content from 0 to 2% in weight regarding the cement weight.

Also in a particular way, additives can be added to modify the rheology of cement paste selected from:

-　dispersants, such as: polyacrylic/polymetacrylic acids and its respective salts; polycarboxylates and modified polycarboxylic ethers; lignonaphthalene- and sulfonated melamine; among others - in content from 0 to 5% in weight regarding the cement weight;
-　plastifyers, such as: oxide, hydroxide, carbon and/or calcium chloride among others, in content from 0 to 10% in weight relating to cement weight;
-　thickening agents, such as: amorphous silica with high specific superficial area; cellulose polymeric, such as hydroxyethylcellulose and carboxymethyl cellulose; in addition to suspensions of collagen and/or chitosan, among others - in content from 0 to 30% in weight relating to cement weight.

In a second aspect, this invention refers to calcium silicate-based nanometric crystals obtained according to the process of this invention, wherein the dimension of the referred crystals varies from 10 and 100. The referred crystals assume an acicular habit or glycerin concentration in wetting water of cement between 0.5% and 2.5% of cement weight - provoking expansion in the cement material of up to 5% in volume - or assume the lamellar habit for glycerin concentration in wetting water of cement from 7.5% to 12.5% in cement weight, provoking an expansion in cement material over 5% in volume.

In a third aspect, said crystals can be used in the covering of petrochemical wells, in filling of odontological and orthopedical cavities and in the structural elements for civil work, as explained as follows.

Thus, this invention allows the expansion of cement pastes and the increment in mechanical performance, especially the resistance to flexion and the tenacity, what makes it useful for cementation of petrochemical wells. In that case, the cement paste is pumped to fill the annular space between the geological formation and the duct. The nucleation and growth of in situ nanometric crystals to the cement paste by addition of glycerin to wetting water don't interfere in the cement paste rheology, that is, in the past ability to be pumped, or in its compactness, determined by the distribution of size of composition particles. Certainly, the growth of such crystals in the cement paste during the hydration provokes its expansion, essential fact for the effective isolation of the petrochemical well, with the advantage of providing a better adhesion between duct and the cement and between the cement and drilling geological formation. The increase of adhesion is resulted from the cement past confinement against the duct and the geological formation, what allows the appropriate choice between the acicular or lamellar crystals of this technology. In other words, the resistance to the expansion provokes the increase in compacting the cement paste, allowing microscopic emptinesses to be sealed and the primary cementation, in general, is more reliable, besides compensating the internal retraction of the cement past inherent to the hydration reaction.

Therefore, this invention is advantageous in relation to the usual toughening processes for cements used in the covering of petrochemical wells, because: (i) favors similar expansion to MgO, when glycerin is added at the same concentration of 1% in cement weight, however insensitive to temperature, an advantage also on ettringite; (ii) increases the resistance to flexion, unlike salts, and the tenacity of the cement paste, that is, its resistance to crack propagation; (iii) induces the formation of crystals chemically compatible to cement, favoring the adhesion between both, with high aspect relations (up to 100) and, also, nanometric, what differs it under the fracture mechanics prism.

Additionally, the nanometric dimension of those crystals is also advantageous for cementation of points where there is deflection of ducts, common to high-angled and directional wells, since the fluid's pressure loss in such points raises the active tension over the well covering. In that case, the crystals' nanometric dimension provides a larger specific superficial area for surface anchorage and for deflection on the cracks propagation way, with the consequent increment to the growing Curve-R behavior. Besides, the nanometric dimension is opposed to the fall in the resistance to the cement paste flexion, common when conventional fibers are added as toughening. According to fracture mechanic theory, the larger is the addition size, the larger is its tensions concentration and, consequently, lower the resistance to material flexion. Therefore, this invention can contribute significantly to the technological thickening of petrochemical well cementation.

This invention is also adapted for application in civil work. With greater resistance to flexion, structural elements based on Portland cement can become more slender, and also, allow the rational use of the own cement. The option for the crystal type to be nucleated and grown in situ to cement paste along the hydration reaction is due to the chosen conformation technique. Choosing acicular crystals, that is, glycerin concentration in wetting water with 1% in weight in relation to the Portland cement weight, preferentially, allows the conformation techniques maintenance already conventional, whereas choosing lamelar crystals, that is, glycerin concentration in wetting water with 10% in weight in relation to Portland cement weight, preferentially, tends to demand that the conformation takes place in metallic forms, able to restrict excessive expansion from internal growth of crystals in the cement paste, and, with that, raise the conformed structural element compacting. In other words, the choice for lamelar crystals adapts more easily to the conformation process of pre-molded structural elements, already used for industrialization of civil work. Those crystals resist thermal decomposition for temperatures of up to 900°C, as shown in Figure 5, a temperature over the fluency of the own steel that reinforces the cement structural elements and can be a positive factor for structural safety of buildings in case of fires.

Regarding the biomedical applications, the Portland cement owes its use thanks to its biocompatibility, attested by a growing number of scientific articles of the kind. It's understood by biocompatibility, the property presented by a material by not causing inflammatory response through host tissue, or then, when such inflammatory response or eventual tissue necrosis do not lead to pathology development. Among Portland cement applications in the biomedical area, can be mentioned the sealing of endodontics canals roots to which the material compacting is an indispensable parameter, as well as the cavity base preparation for accommodating the chewing tensions transferred by the tooth filling resin. The filling of bone faults is also possible, thanks to the injection of cement pasts, mainly in glycerin presence, among them, the ones arisen from osteoporosis — a disease whose number of patients grows every year due to the society's life expectation increase — and also due to the bone metastasis due to oncological cases, being configured as an alternative to the current polymeric cement (PMMA). In all biomedical applications, the choice of the crystal type for cement toughening, that is, acicular or lamelar, depends on the sanity level of adjacent tissues, since the expansion provided by nucleation and growth of those crystals require a certain bone or tooth mechanical resistance to support it, so that the desired toughening mechanism can be explored. Since there is no ideal single formulation for all applications, this invention shows variations which comprise different expansion demands, with consequent reduction of porosity, and customized mechanical resistance.

Generally, the cement materials according to this invention can be conformed in the same way as the conventional cement materials, they can be poured in molds or cavities, with or without vibration aid, or pumped. On the other hand, the techniques for ceramic materials conformation, such as, for instance, the injection, the extrusion with sliding are also subject to be used for cement materials of this invention. Extrusion is a technique consisting of forcing the passage of a united paste through a cavity whose format defines the final form of the conformed body, being possible, in cases wherein such variation represents an advantage, the displacement of a form by which the stationary material acquires the desired format, a technique also known as extrusion with sliding form. The injection is basically an extrusion extension, since the paste going through the extruding cavity is forced to enter into a matrix where it acquires formats much more complex than the continuous transverse section provided by the extrusion only. Frequently, the use of a conformation technique, particularly, may require adjustments in the cement material rheology, that is, the material capacity to flow, or yet, the different applications intended by this invention may require different times for cement material hardening, what requires the incorporation of appropriate substances. Regarding the cement hardening, some additives known as magpie accelerators can be used, among them the calcium aluminate and the cements formed from it, as well as organic acids, such as citric, tartaric or oxalic, among others, and their respective salts. For the modification of cement pastes rheology, the most used additives are chosen among the following substances: (i) dispersals, such as: polyacrylic/ polymetacrylic acids and their respective salts; polycarboxylates and modified polycarboxylic ethers; ligno -, naphthalene - and sulfonated melamine; among others; (ii) plastifyers, such as: oxide, hydroxide, carbonate and/or chloride of calcium, among others; (iii) espessantes, such as: active amorphous silica with specific superficial high area and/or cellulose polymers, such as hydroxyethylcellulose and carboxymethyl cellulose, besides suspensions of collagen and/or chitosan, among others.

In another aspect, this invention refers to the use of glycerin in nucleation and growth in situ of calcium silicate-based nanometric crystals in cement materials.

The following examples are intended to illustrate aspects of this invention without having, however, any limiting character.

## EXAMPLE 1

Glycerin was added to wetting water to hydrate type CPII-E Portland cement type, in relation to the weight water/cement=0.20, being the glycerin concentration in wetting water equivalent to 1% of Portland cement weight. In the sequence, the humidified cement fills the metallic mold cavity being then compacted with pressure of up to 20MPa, in order to simulate the conditions subject to be found in foreseen applications. After compacted, the cement body is stored in an environment with relative humidity of 95% for 7 days, at the end of this period, its mechanical resistance to flexion in 3 points is evaluated verifying a 100% increase in relation to resistance to flexion of conformed cement bodies flexion without glycerin addition into the wetting water. Analysis by electronic microscopy of fracture surface shows the presence of acicular crystals that grew on the surface of cement grains and under the glycerin action, toughening the cement material by deflection on the crack propagation way and by the fractured surfaces anchorage, as shown in Figure 2. The CPII-E replacement of another cement, the CPV-ARI, known for its high initial mechanical resistance, didn't interfere in the nucleation process and calcium silicate-based acicular nanocrystals and in the respective resulting cement product toughening.

## EXAMPLE 2

Results proportionally similar to Example 1 were obtained when conforming cement bodies wet with water in weight relation of water/cement =0.35, being the liquid glycerin concentration in the wetting water equivalent to 1 % of Portland cement weight, with compacting of up to 1 MPa.

## EXAMPLE 3

Glycerin was added to wetting water to hydrate type CPII-E Portland cement in relation to weight water/cement=0.20 in weight relation, being the glycerin concentration in wetting water equivalent to 10% of Portland cement weight. Following, the humidified cement fills the metallic mold cavity being then compacted with pressure of up to 20MPa, in order to simulate the conditions subject to be found in foreseen applications, having remained in such mold for 7 days. At the end of this period, its mechanical resistance to flexion in 3 points is evaluated, being verified a 200% increase in relation to the resistance to flexion of conformed cement bodies without glycerin addition in wetting water. Analysis by electronic microscopy of fracture surface shows the presence of lamellar crystals that grew on cement grain surface under the glycerin action, toughening the cement material by the deflection on the crack propagation way and by the fractured surface anchorage, as shown in Figure 3. The CPII-E replacement for another cement, the CPV-ARI, known for its high initial mechanical resistance, didn't interfere in the nucleation process and growth of calcium silicate-based lamelar nanocrystals and in the respecting resulting cement product toughening.

## EXAMPLE 4

Results proportionally similar to Example 3 were obtained when conforming cement wet bodies wet with water in relation to weight water/cement=0.35 weight, being the liquid glycerin concentration in wetting water equivalent to 10% of Portland cement weight, with compacting of up to 1 MPa.

## EXAMPLE 5

Portland cement was added to a container having excessive water, in other words, relation water/cement (w/c) over 100, adding glycerin to wetting water in a 10% proportion of cement weight. After 7 days, such suspension was filtered and the solid material analyzed by electronic microscopy, showing the total coverage of cement grains by crystals with lamelar format organized in rosette form, as shown in Figure 4. Those crystals resist to thermal decomposition for temperatures of up to 900°C, according to Figure 5.

A technician will promptly know how to evaluate, through the teaching contained in the text and in the submitted examples, the invention advantages and propose variations and equivalent alternatives of accomplishment, without however departing from the invention scope, as defined in the enclosed claims.

**Claims**

1.  NUCLEATION PROCESS AND IN SITU GROWTH OF CALCIUM SILICATE-BASED NANOMETRIC CRYSTALS IN CEMENT MATERIALS **characterized by** the fact of including glycerin to the cement in concentration from 0.5% to 12.5% in weight, in relation to the cement weight.

2.  PROCESS, according to claim, **characterized by** the fact of mixing an aqueous solution of glycerin to cement, keeping the relation in weight of water to cement between 0.15 and 0.45.

3.  PROCESS, according to one of the claims 1 or 2, **characterized by** the fact that the mixture is subject to the conformation with compacting aid whose pressure may vary from the atmospheric one up to 20MPa.

4.  PROCESS, according to one of the claims 1 to 3, **characterized by** the fact that the cement is of Portland type.

5.  PROCESS, according to one of the claims 1 or 2, **characterized by** the fact that the used glycerin is found in liquid state and at room temperature.

6.  PROCESS, according to one of the claims 1 to 5, **characterized by** the fact that the mixture of glycerin and cement is accomplished by aspersion or dripping of liquid over powder or by the addition of powder to liquid.

7.  PROCESS, according to the claim 6, **characterized by** the fact that it uses aid of mechanical equipment.

8.  PROCESS, according to one of the claims 1 to 7, **characterized by** the fact that the conformation of the obtained cement material is accomplished by pressing, pumping, injection, extrusion , extrusion with sliding forms or the action of pouring in molds or cavities, with or without vibration aid.

9.  PROCESS, according to one of the claims 1 to 8, **characterized by** the fact that additives are added to accelerate the hardening of the selected cement from:

    calcium aluminate and/or its respective cements, in a content from 0 to 30% in weight relating to cement weight; organic acids, such as citric, tartaric or oxalic, among others, and their respective salts, in a content from 0 to 2% in weight relating to cement weight.

10. PROCESS, according to one of the claims 1 to 8, **characterized by** the fact that additive are added to modify the cement paste rheology selected from:

    dispersants, such as: poliacrílico/polimetacrílico acids and its respective salts; polycarboxylates and modified polycarboxylic ethers; ligno-, naftaleno- and melamino-sulfonados; among others — in a content from 0 to 5% in weight relating to cement weight;
    plastifyers, such as: oxide, hydroxide, carbonate and/or calcium chloride, among others, in a content from 0 to 10% in weight relating to the cement weight;
    thickening agents, such as: amorphous silica with specific superficial high area and/or cellulose polymers, such as hydroxyethylcellulose and carboxymethyl cellulose, besides collagen suspensions and/or chitosan, among others, in a content from 0 to 30% in weight relating to cement weight.

11. CALCIUM SILICATE-BAED NANOMETRIC CRYSTALS **characterized by** the fact of being obtained according to the process of claims 1 to 10, wherein the aspect relation of the referred crystals varies between 10 and 100.

12. CRYSTALS, according to claim1, **characterized by** the fact that they assume acicular habit for glycerin concentration in wetting water of the cement between 0.5% and 2.5% of cement weight and they provoke an expansion in the cement material of up to 5% in volume.

13. CRYSTALS, according to claim 1, **characterized by** the fact that assume a lamelar habit for glycerin concentration in the cement wetting water from 7.5% to 12.5% in cement weight and they provoke an expansion in the cement material of, at least, 5% in volume.

14. USE OF CRYSTALS, according to one of the claims 11 to 13, **characterized by** the fact of being in the cement toughening that cover the petrochemical wells, that fill the deontological and orthopedical cavities and that comprise the structural elements for civil work.

**15.** USE OF GLYCERIN **characterized by** the fact of being in the nucleation and in situ growth of calcium silicate-based nanometric crystals in cement materials.

FIG. 1

FIG. 2

FIG. 3

FIG. 4

FIG. 5

# EP 2 695 850 A1

## INTERNATIONAL SEARCH REPORT

| International application No. |
| --- |
| PCT/BR2011/000484 |

**A. CLASSIFICATION OF SUBJECT MATTER**

**B82Y 40/00 (2011.01), C04B 24/02 (2006.01), C08K 5/053 (2006.01), E21D 5/04 (2006.01), A61K 6/06 (2006.01), E04C 3/00 (2006.01)**

According to International Patent Classification (IPC) or to both national classification and IPC

**B. FIELDS SEARCHED**

Minimum documentation searched (classification system followed by classification symbols)

**B82Y 40/00, C04B 24/02, C08K 5/053, E21D 5/04, A61K 6/06, E04C 3/00**

Documentation searched other than minimum documentation to the extent that such documents are included in the fields searched

**Banco de Patentes INPI - BR (SINPI), Portal CAPES**

Electronic data base consulted during the international search (name of data base and, where practicable, search terms used)

**EPODOC, Google Patents, USPTO**

**C. DOCUMENTS CONSIDERED TO BE RELEVANT**

| Category* | Citation of document, with indication, where appropriate, of the relevant passages | Relevant to claim No. |
| --- | --- | --- |
| A | WO 2008128347 A1 (INNOVATIVE BIOCERAMIX INC [CA]) 30 October 2008 (2008-10-30) abstract | 1 a 15 |
| A | US 4015040 A ( NAKAMURA REIKO) 29 March 1977 (1977-03-29) column 1, lines 12-16 and column 2; lines 23-30 | 1 a 15 |
| A | WO 2011110509 A1 ( DRESEN ALEXANDER [DE]) 15 September 2011 (2011-09-15) abstract | 1 a 15 |

| ☐ Further documents are listed in the continuation of Box C. | **X** See patent family annex. |
| --- | --- |

\* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search **15-02-12** | Date of mailing of the international search report **17-02-12** |
| --- | --- |
| Name and mailing address INSTITUTO NACIONAL DA PROPRIEDADE INDUSTRIAL Rua Mayrink Veiga n° 9, 18° andar cep: 20090-050, Centro - Rio de Janeiro/RJ Facsimile No.  +55 21 3037-3663 | Authorized officer **Aline Marta Vasconcelos Loureiro** Telephone No.  +55 21 3037-3493/3742 |

Form PCT/ISA/210 (second sheet) (July 2009)

14

# EP 2 695 850 A1

<table>
<tr><td colspan="2">INTERNATIONAL SEARCH REPORT<br>Information on patent family members</td><td>International application No.<br><br>PCT/BR2011/000484</td></tr>
</table>

| | | | |
|---|---|---|---|
| WO 2008128347 A1 | 2008-10-30 | CN 101668550 A | 2010-03-10 |
| | | US 2008299093 A1 | 2008-12-04 |
| | | EP 2142225 A1 | 2010-01-13 |
| US 4015040 A | 1977-03-29 | DE 2431812 A1 | 1975-01-30 |
| | | GB 1467717 A | 1977-03-23 |
| | | JP 50024329 A | 1975-03-15 |
| | | JP 54020968 B | 1979-07-26 |
| | | JP 1024578 C | 1980-12-18 |
| | | SE 411019 B | 1979-11-26 |
| | | SE 7408417 A | 1975-01-07 |
| WO 2011110509 A1 | 2011-09-15 | None | 2011-09-15 |

Form PCT/ISA/210 (patent family annex) (July 2009)

15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20010004936 A
- US 20010018973 A
- US 7424914 B
- US 5769638 A

### Non-patent literature cited in the description

- **ROSSETTO, H.L. ; DE SOUZA, M.F. ; PANDOLFELLI, V.C.** Chaotropic Substances and their Effects on the Mechanical Strength of Portland Cement-Based Materials. *Mat. Ras.,* 2008, vol. 11, 183-85
- **LOW, N.M.P. ; BEAUDOIN, J.J.** The flexural toughness and ductility of Portland cement-based binders reinforced with wollastonite micro-fibers. *Cem. Concr. Res.,* 1994, vol. 24 (2), 250-58
- **MÜLLER, F.A. ; GBURECK, U. ; KASUGA, T. ; MIZUTANI, Y. ; BARRALET, J.E. ; LOHBAUER, U.** Whisker-reinforced calcium phosphate cements. *J. Am. Ceram. Soc.,* 2007, vol. 90 (11), 3694-97
- **SU, J. ; WANG, Z. ; YONGGANG, Y. ; YONGFA, W. ; CAO, L. ; MA, Y. ; YU, B. ; LI, M.** Nanoporous calcium silicate and PLGA bio-composite for bone repair. *J. Nanomaterials,* 2010, 181429
- **RODRIGUEZ-LORENZO, L.M. ; GARCIA-CARRODEGUAS, R. ; RODRIGUEZ, M.A. ; DE AZA, S. ; JIMÉNEZ, J. ; LÓPEZ-BRAVO, A. ; FERNANDEZ, M. ; SAN ROMÁN, J.** Synthesis, characterization, bioactivity and biocompatility of nanostructured materials based on the wollastonite-poly (ethylmethacrylate-co-vynilpyrrolidone) system. *J. Biomol. Mater. Res. A,* 2008, vol. 88, 53-64
- **LIN, K. ; CHANG, J. ; LU, J.** Synthesis of wollastonite nanowires through hydrothermal microemulsion methods. *Mater. Lett.,* 2006, vol. 60, 3007-10
- **LIN, K. ; CHANG, J. ; CHEN, G. ; RUAN, M. ; NING, C.** Simple method to synthesize single-crystalline ($\beta$-wollastonite nanowires. *J. Crystal Growth,* 2007, vol. 300, 267-71